(19) Europäisches Patentamt
European Patent Office
Office européen des brevets

(11) Numéro de publication: **0 183 591**
**B1**

(12) # FASCICULE DE BREVET EUROPEEN

(45) Date de publication du fascicule du brevet: **10.10.90**

(51) Int. Cl.$^5$: **C 07 C 68/06,** C 07 C 69/96

(21) Numéro de dépôt: **85402158.1**

(22) Date de dépôt: **08.11.85**

(54) **Procédé de préparation des carbonates de bromo-1 éthyle et d'hydrocarbyle et nouveaux carbonates de bromo-1 éthyle et d'hydrocarbyle.**

(30) Priorité: **23.11.84 FR 8417847**

(43) Date de publication de la demande:
**04.06.86 Bulletin 86/23**

(45) Mention de la délivrance du brevet:
**10.10.90 Bulletin 90/41**

(84) Etats contractants désignés:
**CH DE FR GB IT LI SE**

(56) Documents cités:
**FR-A-25 329 33**

(73) Titulaire: **SOCIETE NATIONALE DES POUDRES ET EXPLOSIFS**
**12, quai Henri IV**
**F-75181 Paris Cédex 04 (FR)**

(72) Inventeur: **Malfroot, Thierry**
**17 Chemin des Jardins**
**F-91100 Saintry sur Seine (FR)**
Inventeur: **Piteau, Marc**
**38 avenue de Ballancourt**
**F-91710 Vert-le-Petit (FR)**
Inventeur: **Senet, Jean-Pierre**
**79 rue de la Gare Herbeauvilliers-Buthiers**
**F-77760 La Chapelle la Reine (FR)**

**Le dossier contient des informations techniques présentées postérieurement au dépôt de la demande et ne figurant pas dans le présent fascicule.**

**EP 0 183 591 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

L'invention concerne un nouveau procédé de préparation des carbonates de bromo-1 éthyle et d'hydrocarbyle. L'invention concerne également de nouveaux carbonates de bromo-1 éthyle et d'hydrocarbyle. Si la référence à quelques carbonates alpha-bromés apparait dans un petit nombre de publications seul le carbonate de bromo-1 éthyle et d'éthyle semble avoir fait l'objet d'une description précise. Son obtention ne semble pas aisée. Différents procédés de préparation ont été proposés. L'un d'entre eux consiste à faire réagir un halogénoformiate de bromo-1 éthyle sur l'éthanol selon la réaction:

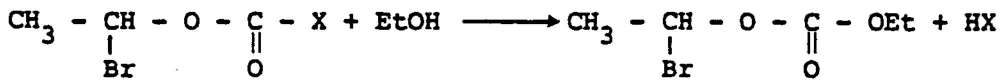

X représente le chlore ou le brome.

La difficulté de ce procédé réside dans l'obtention de l'halogénoformiate bromé de départ

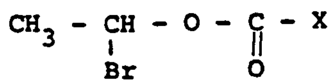

Selon la demande de brevet européen EP 108 547 il est effectué une bromation radicalaire du chloroformiate d'éthyle ou du bromoformiate d'éthyle.

Cette bromation radicalaire présente des inconvénients: les lampes UV employées de préférence aux initiateurs de radicaux libres sont des grands consommateurs d'énergie d'autant plus que les durées de réaction par cette méthode sont importantes. On obntient toujours des sous-produits bromés variés.

Il est également possible de préparer le bromoformiate de bromo-1 éthyle par réaction de l'acétaldéhyde sur le bromophosgène comme décrit dans la demande de brevet français FR 2 532 933. Mais il est alors nécessaire d'utiliser des catalyseurs et le bromophosgène n'est pas un réactif commercialisé. Il est peu stable et très difficile à préparer. On le fabrique par exemple par réaction de l'oxyde de carbone avec le brome mais cette réaction nécessite des installations industrielles très spécifiques.

Un autre procédé de préparation du carbonate de bromo-1 éthyle et d'éthyle consiste à bromer de façon radicalaire le carbonate de diéthyle. On retrouve les mêmes inconvénients des méthodes radicalaires décrites précédemment: obtention de sous-produits, forte demande énergétique. En outre ce procédé ne peut convenir à l'obtention de certains autres carbonates de bromo-1 éthyle car dans certains composés le brome se fixera en plus grande quantité sur l'autre groupe hydrocarboné, par exemple lorsque l'on essaie de fixer le brome sur le carbonate d'éthyle et d'isopropyle on obtient de façon prépondérante le carbonate d'éthyle et de bromo-1 isopropyle.

On peut également utiliser le carbonate de chloro-1 éthyle et d'éthyle comme matière de départ et effectuer une substitution du chlore par le brome au moyen d'un sel organique comme indiqué dans la demande EP n° 108 547. Cette méthode présente également des inconvénients car la réaction de substitution est lente et incomplète.

On obtient par exemple un mélange constitué de 65% seulement de carbonate de bromo-1 éthyle et d'éthyle et de 35% de carbonate de chloro-l'éthyle et d'éthyle (ex 12 de la demande précédemment citée) et la séparation du carbonate α-chloré et du carbonate α-bromé est très difficile comme pour tous les autres carbonates d'alkyle inférieurs.

Depuis longtemps il est connu que la liaison carbone-brome est beaucoup plus facile à couper que la liaison carbone-chlore. Les carbonates α-bromés sont donc particulièrement recherchés par exemple pour l'introduction de groupes carbonates dans une molécule. Il est par conséquent fort intéressant de pouvoir obtenir le maximum d'entre-eux faciliement et avec de bons rendements. Or on constate que les précédents procédés ne donnent pas entière satisfaction.

On a maintenant trouvé un nouveau procédé de préparation des carbonates de bromo-1 éthyle et d'hydrocarbyle.

Selon ce procédé on fait réagir l'acid bromhydrique anhydre sur un carbonate de vinyle et d'hydrocarbyle de formule

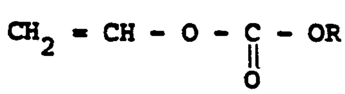

dans laquelle R représente un radical aliphatique en $C_1$ à $C_{12}$ ou cycloaliphatique en $C_5$ à $C_{24}$, saturé, substitué ou non ou un radical aromatique substitué ou non, à une température comprise entre 10° et 100°C.

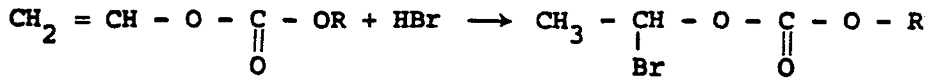

L'addition de l'acide bromhydrique sur divers composés insaturés a fait l'objet de différentes études (voir Houben Weyl "Methoden der organischen Chemie" vol 5/4 pp 107—127). Jusqu'à présent l'addition de cet acide sur une liaison insaturée voisine d'un groupe carbonate n'avait jamais été envisagée. Si elle est relativement aisée sur des composés oléfiniques simples, la présence de groupes fonctionnels sur le carbone porteur de la double liaison rend les résultats de la réaction tout à fait imprévisbles. Ainsi par exemple l'acide bromhydrique décompose l'éther de vinyle et d'éthyle

$$CH_2 = CH—O—CH_2—CH_3$$

immédiatement et on n-observe pas la formation du dérivé bromé. On pouvait penser qu'il en serait de même lors de la fixation de l'acide sur une liaison insaturée voisine d'un oxygène appartenant à un groupe carbonate. Or on a trouvé maintenant de façon surprenante les moyens de réaliser l'addition de l'acide bromhydrique sur les carbonates vinyliques qui de plus est sélective, le brome se fixant uniquement en position alpha.

Les carbonates de vinyle et d'hydrocarbyle utilisés comme matières premières peuvent être obtenus selon les méthodes classiques, par exemple par condensation du chloroformiate de vinyle sur les alcools ou les phénols comme indiqué dans les brevets US 2 377 111, 2 384 143 et 3 903 981, ou par pyrolyse de carbonates selon le procédé décrit dans le brevet US 2 370 549.

Le ou les substituants de R sont généralement choisis dans le groupe comprenant les atomes d'halogène et les radicaux ne portant pas de fonctions sur lesquelles l'acide bromhydrique est susceptible de réagir. Parmi ces derniers le radicaux hydrocarbonés saturés conviennent bien.

L'acide bromhydrique doit être anhydre. Généralement on l'emploie en léger excès, par exemple de l'ordre de 1,05 à 1,4 équivalent par équivalent de carbonate vinylique à traiter.

Selon une version préférée de l'invention on introduit lentement l'acide bromhydrique gazeux dans le milieu réactionnel.

La réaction est réalisée sans solvant.

La température préférée est comprise entre 15 et 60°C. La réaction ne dure généralement que quelques heures. On récupère ensuit les carbonates selon des méthodes classiques généralement par distillation sous pression réduite.

Le procédé selon l'invention est simple. Sa mise en oeuvre ne demande ni installations couteuses, ni une forte dépense d'énergie. Il permet d'obtenir des carbonates de bromo-1 éthyle et d'hydrocarbyle avec une pureté élevée contrairement au procédé par échange chlore-brome dans lequel il se produit un contamination par les carbonates α-chlorés et dans le procédé par bromation radicalaire dans lequel on obtient de nombreux autres produits bromés.

L'invention concerne également les nouveaux carbonates de bromo-1 éthyle et d'hydrocarbyle de formule

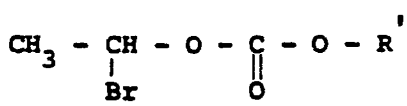

dans laquelle R' est un radical aliphtique en $C_1$ ou en $C_3$ à $C_{12}$ ou cycloaliphatique en $C_5$ à $C_{24}$, saturé, substitué ou non, ou un radical aromatique substitué ou non.

Le ou les substituants de R' sont généralement choisis dans le groupe comprenant les atomes d'halogène et les radicaux ne portant pas de fonctions susceptibles de réagir avec l'acide bromhydrique. Parmi ces derniers les radicaux hydrocarbonés saturés conviennent bien.

L'invention concerne plus particulièrement les carbonates de bromo-1 éthyle et d'isopropyle, de bromo-1 éthyle et de n-octyle, de bromo-1 éthyle et de phényle.

Les carbonates de bromo-1 éthyle et d'hydrocarbyle sont des intermédiaires de synthèse qui peuvent, par exemple, avantageusement remplacer dans de nombreuses réactions leur homologues chlorés moins réactifs ou iodés plus instables.

Une de leurs applications importantes est la modification des fonctions acides carboxyliques de divers composés. La réaction se schématise comme suit:

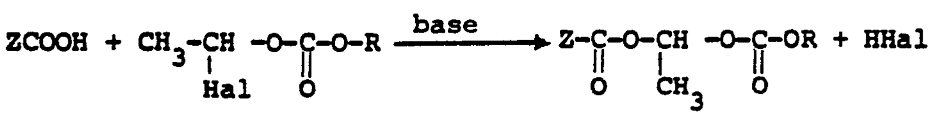


Cette application est en particulier décrite dans les demandes de brevet français 2 532 933 et européen 108 547.

Les carbonates de bromo-1 éthyle peuvent également réagir sur les dérivés de l'acide phosphorique comme indiqué dans la demande allemande 2 628 410:

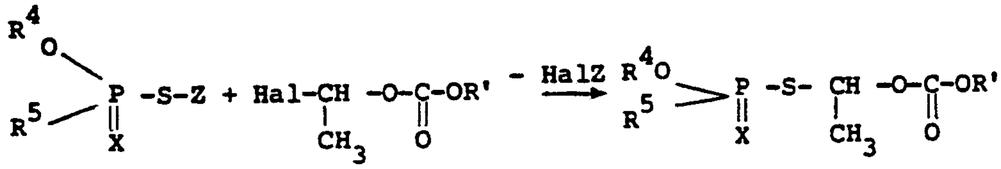


Comme la liaison C—Br est beaucoup plus labile que la liaison C—Cl, on pourra utiliser les carbonates α-bromés en quantité inférieure aux carbonates α-chlorés correspondants. Les conditions réactionnelles seront plus douces et de ce fait on éliminera une certaine dégradation du composé de départ. Les rendements eront plus élevés.

Les carbonates α-bromés sont d'autre part beaucoup plus stables que les carbonates iodés correspondants ce qui permettra lorsqu'ils les remplaceront d'obtenir des produits de pureté plus élevée et en quantité supérieure.

Les exemples suivants sont donnés à titre illustratif:

Example 1-Préparation du carbonate de bromo-1 éthyle et d'éthyle

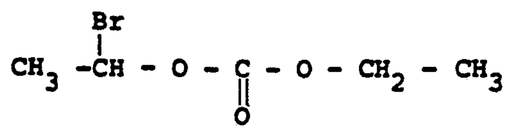

Dans un réacteur muni d'un agitateur, d'un thermomètre, d'un réfrigérant à reflux et d'un tube d'introduction de gaz, on introduit 58 g (0,5 mole) de carbonate de vinyle et d'éthyle.

On fait passer en 1 h 15 min, sous agitation, un courant gazeux de 48 g d'acide bromhydrique anhydre. La température s'élève graduellement de 16 à 35°C.

L'acide bromhydrique en excès est ensuite éliminé par dégazage à l'azote pendant 2 h à 20°C.

Le produit obtenu est purifié par distillation sous pression réduite. On recueille ainsi 75 g (rendement 76%) de carbonate de bromo-1 éthyle et d'éthyle de caractéristiques:

Point d'ébullition (PE): 66—68°C/12 mm Hg

$N_D^{20}$: 1,4410

$d_4^{20}$: 1,4265

RMN$^1$H ($CCl_4$, δ ppm): 1,3 (1, 3H); 1,95 (d, 3H); 4,15 (9, 2H); 6,55 (9, 1H);

IR ν (C=0):1770 $cm^{-1}$.

Exemple 2-Préparation du carbonate de bromo-1 éthyle et d'éthyle

On opère comme à l'exemple 1 mais à partir de 259 g (2,23 moles) de carbonate de vinyle et d'éthyle et de 210 g (2,59 moles) d'acide bromhydrique gazeux anhydre. La durée d'introduction de l'acide bromhydrique est de 8 h, la température évoluant entre 16 et 35°C.

On obtient après traitment du milieu réactionnel comme à l'exemple 1, 378 g de carbonate de bromo-1 éthyle et d'éthyle (rendement 86%)

PE: 75°C/15 mm Hg.

Exemple 3-Préparation du carbonate de bromo-1 éthyle et de n-octyle

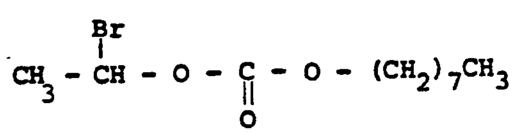

On opère comme à l'exemple 1 à partir de 0,5 mole de carbonate de vinyle et de n-octyle et 0,59 mole d'acide bromhydrique gazeux anhydre.

La durée d'introduction de l'acide bromhydrique est de 3 h, la température étant maintenue à 25°C.

Après distillation sous pression réduite (122°C/4 mm Hg) on recueille le carbonate de bromo-1 éthyle et de n-octyle avec un rendement de 83%.

IR (C=0): 1765 $cm^{-1}$

RMN$^1$H ($CCl_4$, δ ppm): 0,9 (t, 3H); 1,10—1,9 (m, 12H); 2 (d, 3H, J=6Hz); 4,15 (t, 2H, J=6Hz); 6.55 (q, 1H, J=6Hz).

Exemple 4-Préparation du carbonate de bromo-1 éthyle et d'isopropyle

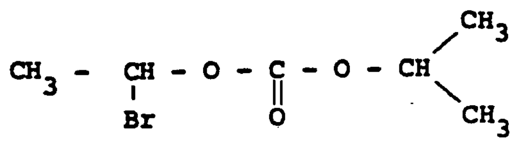

On opère comme à l'exemple 1 avec 0,5 mole de carbonate de vinyle et d'isopropyle et 0,58 mole d'acide bromhydrique gazeux anhydre.

La durée d'introduction de l'acide bromhydrique est de 4 heures, la température étant maintenue à 30°C.

Après distillation sous pression réduite (78°C/16 mm Hg) on recueille le carbonate de bromo-1 éthyle et d'isopropyle avec un rendement de 80%.

IR ν (C=0):1760 $cm^{-1}$

RMN$^1$H ($CCl_4$, δ ppm): 1,3 (d, 6H, J=6Hz); 2 (d, 3H, J=6Hz); 4,9 (sept, 1H, j=6Hz); 6,55 (q, 1H, J=6Hz).

$d_4^{20}$ = 1,3424

$N_D^{20}$ = 1,4372

Exemple 5-Préparation du carbonate de bromo-1 éthyle et de phényle

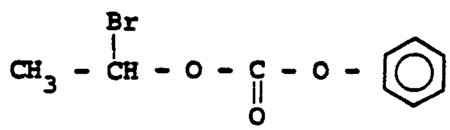

On opère comme à l'exemple 1 avec 0,5 mole de carbonate de vinyle et de phényle et 0,6 mole d'acide bromhydrique gazeux anhydre.

On introduit l'acide bromhydrique pendant 6 h à 20°C puis 2 h à 50°C.

Après distillation sous pression réduite (126°C/5,5 mm Hg) on recueille le carbonate de bromo-1 éthyle et de phényle avec un rendement de 80%.

IR ν (C=0):1765 $cm^{-1}$

RMN$^1$H ($CCl_4$, δ ppm): 2 (d, 3H, J=6Hz); 6,6 (q, 1H, J=6Hz); 7,2 (m 5H).

**Revendications**

1. Procédé de préparation des carbonates de bromo-1 éthyle et d'hyrocarbyle caractérisé en ce que l'on fait réagir l'acide bromhydrique anhydre sur un carbonate de vinyle et d'hydrocarbyle de formule

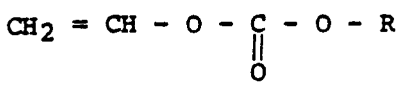

dans laquelle R représente un radical aliphatique en $C_1$ à $C_{12}$ ou cycloaliphtique en $C_5$ à $C_{24}$, saturé, substitué ou non ou un radical aromatique substitué ou non, à une température comprise entre 10° et 100°C.

2. Procédé selon la revendication 1 caractérisé en ce que le ou les substituants de R sont choisis dans le groupe comprenant les atomes d'halogène et les radicaux organiques non porteurs de fonction susceptibles de réagir avec l'acide bromhydrique.

3. Procédé selon la revendication 1 ou 2 caractérisé en ce que R représente l'éthyle, l'isopropyle, le n-octyle ou le phényle.

4. Procédé selon l'une quelconque des revendications précédentes caractérisé en ce que l'acide bromhydrique gazeux est introduit lentement dans le milieu réactionnel.

5. Procédé selon l'une quelconque des revendications précédentes caractérisé en ce que l'acide bromhydrique est utilisé en quantité comprise entre 1,05 et 1,4 équivalent par équivalent de carbonate vinylique à traiter.

6. Procédé selon l'une quelconque des revendications précédentes caractérisé en ce que la réaction est effectuée en l'absence de solvant.

7. Procédé selon la revendication 6 caractérisé en ce que la température de réaction est comprise entre 15° et 60°C.

8. Nouveaux carbonates de bromo-1 éthyle et d'hydrocarbyle de formule:

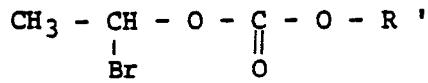

dans laquelle R' est un radical aliphatique en $C_3$ à $C_{12}$ ou cycloaliphtique en $C_5$ à $C_{24}$, saturé, substitué ou non.

9. Carbonates selon la revendication précédente caractérisés en ce que le ou les substituants de R' sont choisis dans le groupe comprenant les atomes d'halogène et les radicaux hydrocarbonés saturés.

10. Carbonate selon la revendication 8 caractérisé en ce que R' est le radical isopropyle.

11. Carbonate selon la revendication 8 caractérisé en ce que R' est le radical cyclohexyle.

**Patentansprüche**

1. Verfahren zur Herstellung von 1-Bromethylhydrocarbylcarbonaten, gekennzeichnet durch Umsetzung von wasserfreier Bromwasserstoffsäure mit einem Vinylhydrocarbylcarbonat der Formel

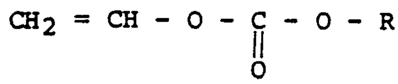

in der R eine ggfs. substituierte gesättigte aliphatische $C_{1-12}$-Gruppe oder cycloaliphatische $C_{5-24}$-Gruppe oder eine ggfs. substituierte aromatische Gruppe bedeutet, bei einer Temperatur von 10 bis 100°C.

2. Verfahren nach Anspruch 1, dadurch gekennzeichnet, daß der oder die Substituenten von R unter Halogenatomen und organischen Gruppen, die keine funktionellen Gruppen tragen, die mit Bromwasserstoffsaüre reagieren könnten, ausgewählt sind.

3. Verfahren nach Anspruch 1 oder 2, dadurch gekennzeichnet, daß R Ethyl, Isopropyl, n-Octyl oder Phenyl darstellt.

4. Verfahren nach einem der vorhergehenden Ansprüche, dadurch gekennzeichnet, daß die gasförmige Bromwasserstoffsaüre langsam in das Reaktionsmedium eingeführt wird.

5. Verfahren nach einem der vorhergehenden Ansprüche, dadurch gekennzeichnet, daß die Bromwasserstoffsäure in einer Menge von 1,05 bis 1,4 Äquivalent pro Äquivalent umzusetzendes Vinylcarbonat eingesetzt wird.

6. Verfahren nach einem der vorhergehenden Ansprüche, dadurch gekennzeichnet, daß die Reaktion in Abwesenheit von Lösungsmittel durchgeführt wird.

7. Verfahren nach Anspruch 6, dadurch gekennzeichnet, daß die Reaktionstemperatur im Bereich von 15 bis 60°C liegt.

8. Neue 1-Bromethylhydrocarbylcarbonate der Formel

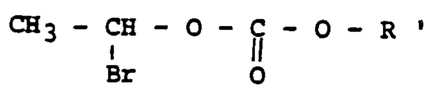

in der R' eine gesättigte und ggfs. substituierte aliphatische $C_{3-12}$-Gruppe oder cycloaliphatische $C_{5-24}$-Gruppe bedeutet.

9. Carbonate nach Anspruch 8, dadurch gekennzeichnet, daß der oder die Substituenten R' unter Halogenatomen und gesättigten Kohlenwasserstoffgruppen ausgewählt sind.

10. Carbonat nach Anspruch 8, dadurch gekennzeichnet, daß R' Isopropyl ist.

11. Carbonat nach Anspruch 8, dadurch gekennzeichnet, daß R' Cyclohexyl ist.

**Claims**

1. Process for the preparation of 1-bromoethyl hydrocarbonyl carbonates, characterized in that anhydrous hydrobromic acid is reacted with a vinyl hydrocarbonyl carbonate of formula

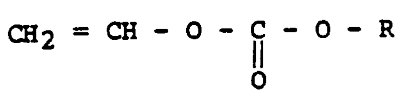

in which R denotes a $C_1$—$C_{12}$ aliphatic or $C_5$—$C_{24}$ alicyclic radical, saturated, substituted or otherwise, or an aromatic radical, substituted or otherwise, at a temperature of between 10° and 100°C.

2. Process according to Claim 1, characterized in that the substituent or the substituents in R are chosen from the group comprising halogen atoms and organic radicals not bearing any functional group liable to react with hydrobromic acid.

3. Process according to Claim 1 or 2, characterized in that R denotes ethyl, isopropyl, n-oxtyl or phenyl.

4. Process according to any one of the preceding claims, characterized in that gaseous hydrobromic acid is introduced slowly into the reaction medium.

5. Process according to any one of the preceding claims, characterized in that hydrobromic acid is employed in a quantity of between 1.05 and 1.4 equivalents per equivalent of vinyl carbonate to be treated.

6. Process according to any one of the preceding claims, characterized in that the reaction is carried out in the absence of solvent.

7. Process according to Claim 6, characterized in that the reaction temperature is between 15° and 60°C.

8. New 1-bromoethyl hydrocarbonyl carbonates of formula:

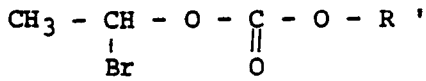

in which R' is a $C_3$—$CC_{12}$ aliphatic or $C_5$—$C_{24}$ alicyclic radical, saturated, substituted or otherwise.

9. Carbonates according to the preceding claim, characterized in that the substituent or subsituents in R' are chosen from the group comprising halogen atoms and saturated hydrocarbon radicals.

10. Carbonate according to Claim 8, characterized in that R' is the isopropyl radial.

11. Carbonate according to Claim 8, characterized in that R' is the cyclohexyl radical.